# EUROPEAN PATENT APPLICATION

(11) **EP 1 475 177 A1**
(43) Date of publication of application: **10.11.2004**
(21) Application number: 04010549.6
(22) Date of filing: 04.05.2004
(51) Int. Cl.: B23K 1/00, H01R 4/02, H05K 3/12

(54) **Apparatus and method for forming solder wicking prevention zone and electronic part**

(30) Priority: 09.05.2003 JP 2003131186; 03.03.2004 JP 2004059793
(71) Applicant: Murata Co., Ltd., Kobe-shi Hyogo-ken (JP); MORIMURA BROS., Inc.,, Minato-ku Tokyo (JP)
(72) Inventor: Kato, Kazuhiko, Takasago-shi Hyogo-ken (JP); Yanagida, Satoshi, Ito-shi Shizuoka-ken (JP); Sato, Osamu, Tokyo (JP); Kondo, Mineo, Chigasaki-shi Kanagawa-ken (JP)
(74) Representative: Dreiss, Fuhlendorf, Steimle & Becker

(57) **Abstract**

The present invention provides an apparatus (30) for forming a solder wicking prevention zone operating when a connector member with a connector is processed using molten solder, to hinder the solder from spreading to a contact site of the connector in a wettable manner. The apparatus includes a main forming agent tank (34) which stores a main forming agent used to form the solder wicking prevention zone, an ejection nozzle (38), in particular an ink jet printer, which ejects the main forming agent stored in the main forming agent tank (34), and a direction control section (46) which controls an ejection direction of the main forming agent ejected by the ejection nozzle (38). The direction control section (46) controls the ejection direction of the main forming agent ejected by the ejection nozzle (38), so as to apply the main forming agent to a predetermined position on the connector member to form the solder wicking prevention zone.

## Description

The present invention relates to an apparatus and method for forming a solder wicking prevention zone, and an electronic part, and for example, to an apparatus and method for forming a solder wicking prevention zone that operates when a joining process using molten solder (hereinafter referred to as a "molten solder process") is executed on an electronic part comprising an electrical contact, for example, a connector member, an IC lead frame, a resistance chip, a film carrier tape, or a capacitor, to inhibit the solder from spreading to a contact site in a wettable manner, as well as an electronic part on which the solder wicking prevention zone is formed.

Electronic parts normally comprising an electrical contact include a connector member, an IC lead frame, a resistance chip, a film carrier tape, and a capacitor. Of these electronic parts, the connector member will be described below as a typical example. The connector member is generally manufactured through the steps described below.

That is, a process of manufacturing a connector member comprises a first step of pressing mainly a copper alloy material into a predetermined shape, a second step of plating the electronic part, a third step of insert-molding the electronic part, and a fourth step of assembling the electronic part into a finished product. The present invention relates to improvement of the plating technique used in the second step.

There are a method of individually punching pressed copper alloy materials and barrel-plating each of these members and what is called a continuous plating method of coiling a pressed copper alloy material and subsequently continuously feeding and plating the coiled material. The present invention can be favorably used for the continuous plating but is applicable to the barrel plating.

With the continuous plating method, first, a material is subjected to a pre-process such as degreasing or activation. The material is then entirely plated with nickel. Then, a contact site is partly plated with gold, while a soldered site is plated with preliminary solder. Another continuous plating method comprises plating the entire surface of the material with nickel, then flashing the entire surface so as to form a thin gold layer, and then continuously adding a number of gold layers to the material which are sufficient to produce a contact effect required for the contact site.

In the fourth step, the material is joined to other parts by soldering to obtain a finished product. However, during the soldering, the quality problem described below may occur, thus making it essential to provide a solder wicking prevention zone. That is, the surface plated with solder and gold has a high affinity for molten solder and is easy to wet. Thus, the molten solder may spread to a gold plated area of the contact site in a wettable manner. This may disadvantageously impair the function of the gold plated area to act as a contact. This is why a "solder wicking prevention zone" is commonly provided in order to prevent the wettable spreading of the molten solder.

The solder wicking prevention zone must be provided, using certain means, with an unwettable material that does not have an affinity for the molten solder. The methods described below have hitherto been used. One of them is the utilization of a nickel plating film. The other is to apply a material to the solder prevention zone which hinders the wetting of solder. However, with either of the two methods, it is very complicated and is not easy to form the solder prevention zone, which is at most 0.5 mm in width.

After the entire surface has been plated with nickel, the area to be formed into the solder wicking prevention zone may be masked so as not to generate solder plating or a gold plating film in this area (this method will hereinafter be referred to as a "masking method"). Alternatively, a plating liquid level may be controlled to expose the area to be formed into the solder wicking prevention zone, to the air. Then, the area requiring a plating film may be plated with solder or gold, so as to leave nickel in the solder wicking prevention zone (this method will hereinafter be referred to as a "liquid level control method"). Another method is to remove, after solder or gold plating, the solder or gold plating from the area to be formed into the solder wicking prevention zone, thus exposing the underlying nickel plated portion (this method will hereinafter be referred to as a "removal method") .

There are two masking methods as described below. One of them is to continuously press, during a solder or gold plating process, a belt-like shielding material against a processed material synchronously with the speed of the processed material. The other is to continuously apply a masking material at least before a solder or gold plating process.

There are also two removal methods as described below. One of them is to apply a masking agent to the entire area except for a site to be removed and use an appropriate agent to etch the site. The other is to irradiate the site with a laser beam to evaporate and remove it.

However, the conventional manufacture of connector products has the problems described below.

The size and pitch of connector products has been increasingly reduced. The width of the solder wicking prevention zone has recently been reduced to 0.5 mm. There is also a strong demand for a further reduction in width.

The conventional masking method and liquid level control method have reached their limits in connection with the maintenance of the above accuracy. Under these circumstances, the laser beam irradiation method, belonging to the removal method, is promising.

However, although this technique is useful because it enables a very small area to be continuously irradiated with a laser beam while precisely controlling the positional accuracy, it has not been put to practical use for the following reason. The solder plating or the gold plating film must be vaporized in the air until the underlying nickel plating is exposed. Consequently, the target area may be exposed to a high temperature, though it is very small.

Gold has a melting point of 1,050°C. The melting point of solder plating varies with its type; Sn-10Pb (solder composed of a base material of Sn to which 10 wt% of Pb is added), which is commonly used, has a melting point of 217°C. A gold plating layer is thin but has a high melting point. Solder plating has a low melting point but is about 5 µm in thickness. Accordingly, the desired physical properties of electronic parts such as connector products may be thermally impaired. No proper solutions to this problem have been found out yet.

The above described problems with the manufacture of electronic parts will be summarized below.
1) Connectors are used as electrical contacts and are repeatedly installed and removed. Accordingly, the degradation of the spring characteristic or mechanical intensity of the connector is counted as an unacceptable important characteristic. Irradiation with a laser beam may impair the properties of areas close to the contact.
2) The gold or solder plating on the nickel plating film reaches its melting point or higher under heat. Accordingly, interdiffusion is unavoidable. This may cause a fragile inter-metal compound to be generated to reduce the mechanical strength of this structure. In addition, compared to the nickel plating film alone, the structure has an insufficient wettability to provide a solder wicking prevention zone.
3) A coherent and highly linear laser beam is ineffective on a surface that does not face the beam, such as a surface of a curved body or a punched press ruptured surface because it does not impinge directly on such a surface. The solder wicking prevention zone is insufficiently effective when provided only on part of the target. Ideally, the solder wicking prevention zone is provided all along the periphery of a predetermined position on the electronic part. With the removal process with a laser beam, a solder wicking prevention zone cannot be provided all along the periphery of the connector using a single apparatus.
4) Laser beam irradiation apparatuses are very expensive and are too large to be placed on a continuous plating line for connectors and used as online apparatuses. Accordingly, processing on another line is required, thus making it impossible to give advantages offsetting the high cost.

The present invention is provided in view of these circumstances. It is a first object of the present invention to provide an apparatus and method for forming a solder wicking prevention zone that reliably hinders solder wicking without impairing the properties of an electronic part, the apparatus and method enabling the solder wicking prevention zone to be inexpensively formed.

It is a second object of the present invention to provide an electronic part provided with a solder wicking prevention zone that effectively prevents solder wicking.

To accomplish these objects, the present invention provides the means described below.

According to a first aspect of the present invention, there is provided an apparatus for forming a solder wicking prevention zone operating when a connector member with a connector is processed using molten solder, to hinder the solder from spreading to a contact site of the connector in a wettable manner, the apparatus comprising storage means for storing a forming agent used to form the solder wicking prevention zone, ejecting means for ejecting the forming agent stored in the storage means, and control means for controlling an ejection direction of the forming agent ejected by the ejecting means.

The control means controls the ejection direction of the forming agent ejected by the ejecting means, so as to apply the forming agent to a predetermined position on the connector member to form the solder wicking prevention zone.

The use of the above means enables the solder wicking prevention zone to be formed by utilizing the principle of ink jet printers to eject the forming agent used to form the solder wicking prevention zone, so as to apply it to the predetermined position on the connector member.

The apparatus enables a solder wicking prevention zone of a desired size to be easily formed using an accurate printing function inherently provided in ink jet printers and without heating the connector member. Further, since the ink jet printer is not as large as laser beam irradiation apparatuses but is economical, it can be easily installed on an existing line and inexpensively implemented.

According to a second aspect of the present invention, in the apparatus for forming a solder wicking prevention zone according to the first aspect, a plurality of the ejecting means and a plurality of the corresponding control means are arranged to surround the predetermined position on the connector member so that the control means corresponding to each ejecting means can control the ejection direction of the forming agent ejected by the ejecting means, thus allowing the forming agent to be applied to the predetermined position on the connector member.

The above means enables the solder wicking prevention zone to be formed along the outer periphery of the connector member in a short time.

According to a third aspect of the present invention, in the apparatus for forming a solder wicking prevention zone according to the first and second aspects, a ultraviolet cured resin is used as the forming agent, and the apparatus further comprises ultraviolet irradiation means for irradiating the predetermined position coated with the forming agent with ultraviolet rays.

The above means enables the solder wicking prevention zone to be stabilized in a short time by applying the forming agent consisting of the ultraviolet cured resin and subsequently irradiating the ultraviolet cured resin with ultraviolet rays to harden the resin. As a result, the solder wicking prevention zone can be more efficiently formed.

According to a fourth aspect of the present invention, there is provided an apparatus for forming a solder wicking prevention zone operating when a connector member with a connector is processed using molten solder, to hinder the solder from spreading to a contact site of the connector in a wettable manner, the apparatus comprising first storage means for storing a main forming agent used to form the solder wicking prevention zone, second storage means for storing an additive added to the main forming agent, first ejecting means for ejecting the main forming agent stored in the first storage means, second ejecting means for ejecting the additive stored in the second storage means, and control means for controlling ejection directions of the forming agent and additive ejected by the first and second ejecting means, respectively.

The control means control the ejection directions of the forming agent and additive ejected by the first and second ejecting means, respectively, so as to apply the forming agent and the additive to a predetermined position on the connector member to form the solder wicking prevention zone.

The use of the above means enables the apparatus to be used in various manners depending on applications by using, for example, a polymerization initiator, a polymerization regulator, a viscosity regulator, a coloring agent, or the like as the additive.

For example, the nature of the solder wicking prevention zone can be arbitrarily regulated by using a polymerization initiator, a polymerization regulator, or a viscosity regulator, or their appropriate mixture as the additive. Using a coloring agent as the additive enables the finished solder wicking prevention zone to be checked. This makes it possible to reduce the time required for inspections.

According to a fifth aspect of the present invention, in the apparatus for forming a solder wicking prevention zone according to the fourth aspect, a plurality of the ejecting means and a plurality of the corresponding control means are arranged to surround the predetermined position on the connector member so that the control means corresponding to the respective ejecting means can control the ejection directions of the forming agent and additive ejected by the ejecting means, thus allowing the forming agent and the additive to be applied to the predetermined position on the connector member.

The above means enables the apparatus to be used in various manners depending on applications by using, for example, a polymerization initiator, a polymerization regulator, a viscosity regulator, a coloring agent, or the like as the additive. Furthermore, the solder wicking prevention zone can be formed along the outer periphery of the connector member in a short time.

According to a sixth aspect of the present invention, there is provided a method for forming a solder wicking prevention zone using an ink jet printer comprising storage means for storing ink, ejecting means for ejecting the ink stored in the storage means, and control means for controlling an ejection direction of ink ejected by the ejecting means, the solder wicking prevention zone operating when a connector member with a connector is processed using molten solder, to hinder the solder from spreading to a contact site of the connector in a wettable manner, the method comprising storing a forming agent used to form the solder wicking prevention zone, in the storage means, instead of the ink, allowing the ejecting means to eject the forming agent stored in the storage means, and allowing the control means to control an ejection direction of the forming agent ejected by the ejecting means, so as to apply the forming agent to a predetermined position on the connector member to form the solder wicking prevention zone.

The use of the above means enables a solder wicking prevention zone to be formed by utilizing the principle of ink jet printers to eject the forming agent used to form the solder wicking prevention zone, so as to apply it to the predetermined position on the connector member.

The method enables a solder wicking prevention zone of a desired size to be easily formed using an accurate printing function inherently provided in ink jet printers and without heating the connector member. Further, since the ink jet printer is not as large as laser beam irradiation apparatuses but is economical, it can be easily installed on an existing line and inexpensively implemented.

According to a seventh aspect of the present invention, in the method for forming a solder wicking prevention zone according to the sixth aspect, a plurality of the ejecting means and a plurality of the corresponding control means are arranged to surround the predetermined position on the connector member so that the control means corresponding to each ejecting means can control the ejection direction of the forming agent ejected by the ejecting means, thus allowing the forming agent to be applied to the predetermined position on the connector member.

The above means enables the solder wicking prevention zone to be formed along the outer periphery of the connector member in a short time.

According to an eighth aspect of the present invention, in the method for forming a solder wicking prevention zone according to the sixth or seventh aspect, a ultraviolet cured resin is used as the forming agent, and the ultraviolet cured resin is cured by irradiating the predetermined position coated with the forming agent with ultraviolet rays.

The above means enables the solder wicking prevention zone to be stabilized in a short time by applying the forming agent consisting of the ultraviolet cured resin and subsequently irradiating the ultraviolet cured resin with ultraviolet rays to harden the resin. As a result, the solder wicking prevention zone can be more efficiently formed.

According to a ninth aspect of the present invention, there is provided a method for forming a solder wicking prevention zone using an ink jet printer comprising first storage means for storing ink, second storage means for storing an additive added to the ink, first ejecting means for ejecting the ink stored in the first storage means, second ejecting means for ejecting the additive stored in the second storage means, and control means for controlling ejection directions of the ink and additive ejected by the first and second ejecting means, respectively, the solder wicking prevention zone operating when a connector member with a connector is processed using molten solder, to hinder the solder from spreading to a contact site of the connector in a wettable manner, the method comprising storing a main forming agent used to form the solder wicking prevention zone, in the first storage means, instead of the ink, and storing an additive added to the main forming agent, in the second storage means, allowing the first ejecting means to eject the main forming agent stored in the first storage means, allowing the second ejecting means to eject the additive stored in the second storage means, and allowing the control means to control ejection directions of the forming agent and additive ejected by the first and second ejecting means, respectively, so as to apply the forming agent and the additive to a predetermined position on the connector member to form the solder wicking prevention zone.

Accordingly, as in the case of the apparatus for forming a solder wicking prevention zone according to the fourth aspect, the method for forming a solder wicking prevention zone according to the ninth aspect can be used in various manners depending on applications by using, for example, a polymerization initiator, a polymerization regulator, a viscosity regulator, a coloring agent, or the like as the additive.

According to a tenth aspect of the present invention, in the method for forming a solder wicking prevention zone according to the ninth aspect, a plurality of the ejecting means and a plurality of the corresponding control means are arranged to surround the predetermined position on the connector member so that the control means corresponding to the respective ejecting means can control the ejection directions of the forming agent and additive ejected by the ejecting means, thus allowing the forming agent and the additive to be applied to the predetermined position on the connector member.

The above means enables the method to be used in various manners depending on applications by using, for example, a polymerization initiator, a polymerization regulator, a viscosity regulator, a coloring agent, or the like as the additive. Furthermore, the solder wicking prevention zone can be formed along the outer periphery of the connector member in a short time.

An electronic part according to an eleventh aspect comprises a solder wicking prevention zone that hinders solder from spreading in a wettable manner during a molten solder process, the solder wicking prevention zone being provided by applying a material that is not wet with the solder, to the electronic part.

The above means enables the provision of an electronic part provided with a solder wicking prevention zone that effectively prevents solder wicking.

According to a twelfth aspect of the present invention, in the electronic part according to the eleventh aspect, the material that is not wet with the solder contains at least one or more of resin, ceramic, carbon, and glass.

The above means enables the provision of an electronic part provided with a solder wicking prevention zone that effectively prevents solder wicking.

According to a thirteenth aspect of the present invention, in the electronic part according to the eleventh or twelfth aspect, the solder wicking prevention zone is colored differently from a coated surface so as to be discriminated from the coated surface.

The above means makes it possible to provide an electronic part provided with a solder wicking prevention zone that effectively prevents solder wicking. Moreover, it is possible to easily discriminate electronic parts provided with the solder wicking prevention zone from electronic parts not provided with the solder wicking prevention zone. Furthermore, electronic parts can be discriminated from one another by differently coloring their solder wicking prevention zones.

According to a fourteenth aspect of the present invention, there is provided an apparatus for forming a solder wicking prevention zone operating when an electronic part is processed using molten solder, to hinder the solder from spreading out from a joined site of the electronic part in a wettable manner, the apparatus comprising storage means for storing a forming agent used to form the solder wicking prevention zone, ejecting means for ejecting the forming agent stored in the storage means, control means for controlling an ejection amount and an ejection speed of the forming agent ejected by the ejecting means, and curing means for curing the forming agent ejected by the ejecting means and applied to a predetermined position on the electronic part. Examples of the curing means include a method of thermally curing the forming agent, a method of curing the forming agent by burning and decomposition, and a method of curing the forming agent by irradiating it with light such as ultraviolet rays or an electron beam.

The above means enables the electronic part to be provided with a solder wicking prevention zone that effectively prevents solder wicking.

According to a fifteenth aspect of the present invention, in the apparatus according to the fourteenth aspect, a plurality of the ejecting means are arranged to surround the predetermined position on the electronic part so that the control means can control the ejection amount and ejection speed of the forming agent ejected by each ejecting means, thus allowing the forming agent to be applied to the predetermined position.

The above means enables the electronic part to be reliably provided with a solder wicking prevention zone that effectively prevents solder wicking.

According to a sixteenth aspect of the present invention, in the apparatus according to the fourteenth or fifteenth aspect, a ultraviolet cured resin is used as the forming agent, and the apparatus further comprises ultraviolet irradiation means for irradiating the forming agent applied to the predetermined position with ultraviolet rays.

The above means enables the electronic part to be quickly provided with a solder wicking prevention zone that effectively prevents solder wicking.

According to a seventeenth aspect of the present invention, in the apparatus according to any of the fourteenth to sixteenth aspects, a dispenser is used as the ejecting means.

The above means enables the use of the simple and inexpensive apparatus to provide the electronic part with a solder wicking prevention zone that effectively prevents solder wicking so that the solder wicking prevention zone is accurately positioned.

According to an eighteenth aspect of the present invention, there is provided a method for forming a solder wicking prevention zone operating when an electronic part is processed using molten solder, to hinder the solder from spreading out from a joined site of the electronic part in a wettable manner, the method comprising ejecting a predetermined amount of forming agent used to form the solder wicking prevention zone, from a nozzle in a dispenser at a predetermined ejection speed, to apply the forming agent to a predetermined position on the electronic part, and curing the applied forming agent to form the solder wicking prevention zone.

The above means enables the use of the simple and inexpensive apparatus to provide the electronic part with a solder wicking prevention zone that effectively prevents solder wicking so that the solder wicking prevention zone is accurately positioned.

According to a nineteenth aspect of the present invention, in the method according to an eighteenth aspect, a ultraviolet cured resin is used as the forming agent, and the forming agent applied to the predetermined position is irradiated with ultraviolet rays and thus harden.

The above means enables the use of the simple and inexpensive apparatus to quickly provide the electronic part with a solder wicking prevention zone that effectively prevents solder wicking so that the solder wicking prevention zone is accurately positioned.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a sectional view showing an example of a connector portion comprising a solder wicking prevention zone;
FIG. 2 is a plane view showing an example of a base material composed of, for example, a copper alloy;
FIG. 3 is a sectional view of connector portions and a planar portion, showing an example of a method of manufacturing a connector member;
FIG. 4 is a sectional view showing an example of a connector portion not comprising a solder wicking prevention zone (solder has not spread to a gold plated contact site);
FIG. 5 is a sectional view showing the example of the connector portion not comprising the solder wicking prevention zone (solder has spread to the gold plated contact site);
FIG. 6 is a functional block diagram showing an example of the configuration of an apparatus for forming a solder wicking prevention zone to which apparatus a method for forming a solder wicking prevention zone according to a first embodiment is applied;
FIG. 7 is a schematic diagram showing an example of arrangement of the components of the apparatus for forming a solder wicking prevention zone to which apparatus the method for forming a solder wicking prevention zone according to the first embodiment is applied;
FIG. 8 is a conceptual drawing showing an area coated with a main forming agent from one ejection nozzle;
FIG. 9 is a flowchart showing an operation of the apparatus for forming a solder wicking prevention zone to which apparatus the method for forming a solder wicking prevention zone according to the first embodiment is applied;
FIG. 10 is a sectional view showing an example of a connector portion comprising a solder wicking prevention zone;
FIG. 11 is a partial sectional view showing an example of a solder wicking prevention zone provided on a connector portion (the solder wicking prevention zone is provided directly on a base material);
FIG. 12 is a partial sectional view showing an example of a solder wicking prevention zone provided on a connector portion (the solder wicking prevention zone is provided on gold flash plating);
FIG. 13 is a partial sectional view showing an example of a solder wicking prevention zone provided on a connector portion (solder passes over the solder wicking prevention zone);
FIG. 14 is a partial sectional view showing an example of a solder wicking prevention zone provided on a connector portion (the solder does not pass over the solder wicking prevention zone);
FIG. 15 is sectional view showing the connector portions and planar portion, showing the example of the method for manufacturing connector portions (after processing);
FIG. 16 is a sectional view showing an example of a connector portion not comprising a solder wicking prevention zone (solder has not spread to a gold plated contact site);
FIG. 17 is a sectional view showing the example of the connector portion not comprising the solder wicking prevention zone (the solder has spread to the gold plated contact site);
FIG. 18 is a functional block diagram showing an example of the configuration of an apparatus for forming a solder wicking prevention zone to which apparatus a method for forming a solder wicking prevention zone according to a second embodiment is applied;
FIG. 19 is a schematic diagram showing an example of the shape of the tip of a nozzle;
FIG. 20 is a schematic diagram showing an example of a multi-nozzle;
FIG. 21 is a conceptual drawing showing the positional relationship between a nozzle and a connector portion (the tip of the nozzle is slightly separated from the surface of the connector portion);
FIG. 22 is a conceptual drawing showing the positional relationship between the nozzle and the connector portion (the tip of the nozzle is pressed against the connector portion);
FIG. 23 is a conceptual drawing showing the positional relationship between the nozzle and the connector portion (the tip of the nozzle is completely separated from the surface of the connector portion); and
FIG. 24 is a conceptual drawing showing an example of arrangement of nozzles used if a forming agent is shot at the connector portion.

Embodiments of the present invention will be described below with reference to the drawings.

### (First Embodiment)

A first embodiment of the present invention will be described below with reference to the drawings.

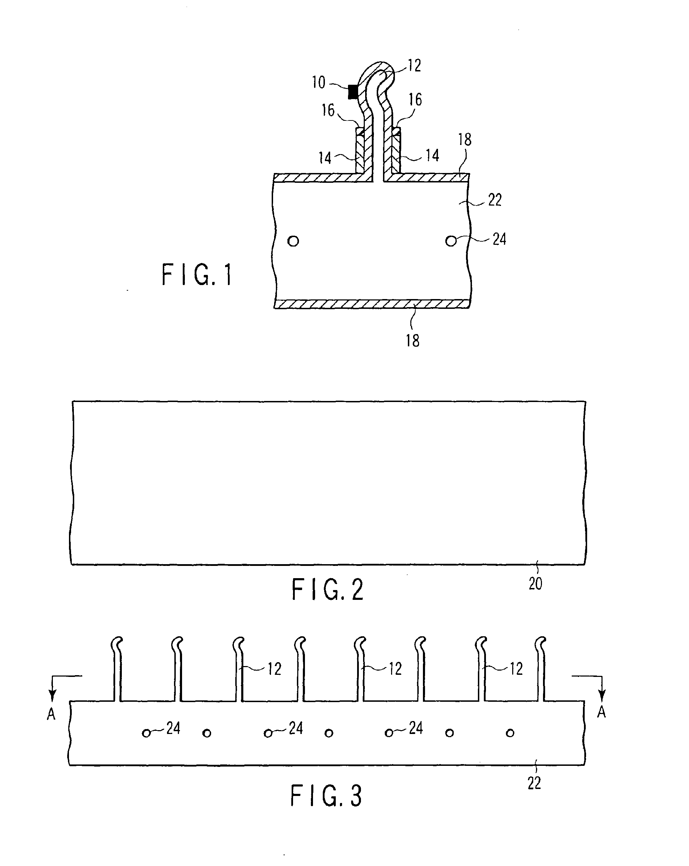

With an apparatus for forming a solder wicking prevention zone to which apparatus a method for forming a solder wicking prevention zone according to the first embodiment is applied, a connector portion 12 comprising, for example, a gold plated contact site 10 is formed with a solder wicking prevention zone 16 that operates if a molten solder 14 is applied to the connector portion 12, to hinder the molten solder 14 from rising and spreading to the gold plated contact site 10 in a wettable manner as shown in FIG. 1. The connector portion 12 is composed of, for example, a copper alloy. The entire surface of the connector portion 12 is covered with nickel plating 18.

With reference to FIGS. 2 and 3, description will be given of a method of forming the connector portion 12.

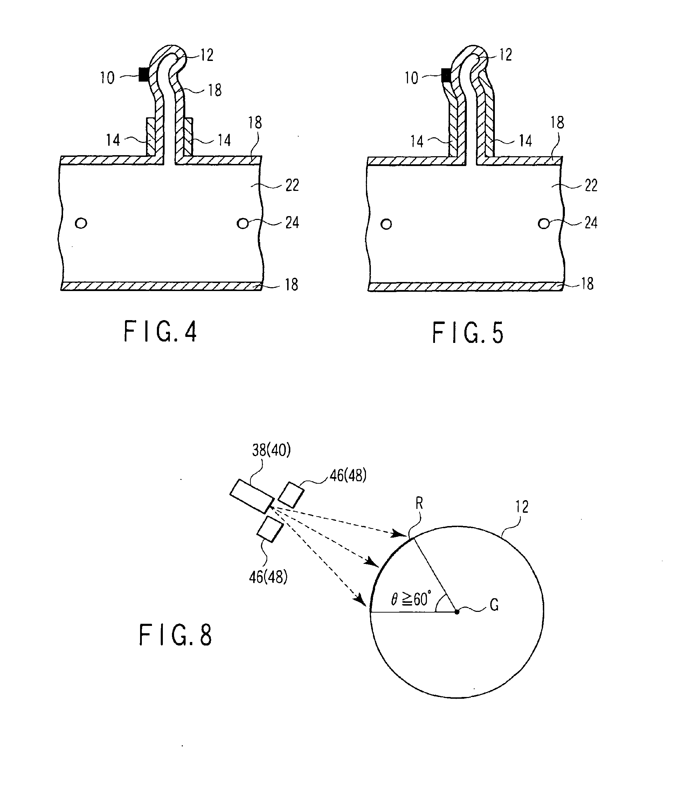

First, the nickel plating 18 is applied so that the entire surfaces of the connector portion 12 and planar portion 22 are converted with the nickel plating 18. Moreover, the connector portion 12 is partly plated with gold to form the gold plated contact site 10. An outer peripheral surface of a leg portion of the connector portion 12 is connected to another part using the molten solder 14. FIG. 4 shows, by way of example, the connector portion 12 integrated with the planar portion 22. However, the nickel plating 18 is applied even when the connector portion 12 is joined to another part.

In this case, without the solder wicking prevention zone 16, the molten solder 14 spreads to the height of the contact site 10 in a wettable manner as shown in FIG. 5. Consequently, the contact site 10 may not function as a contact.

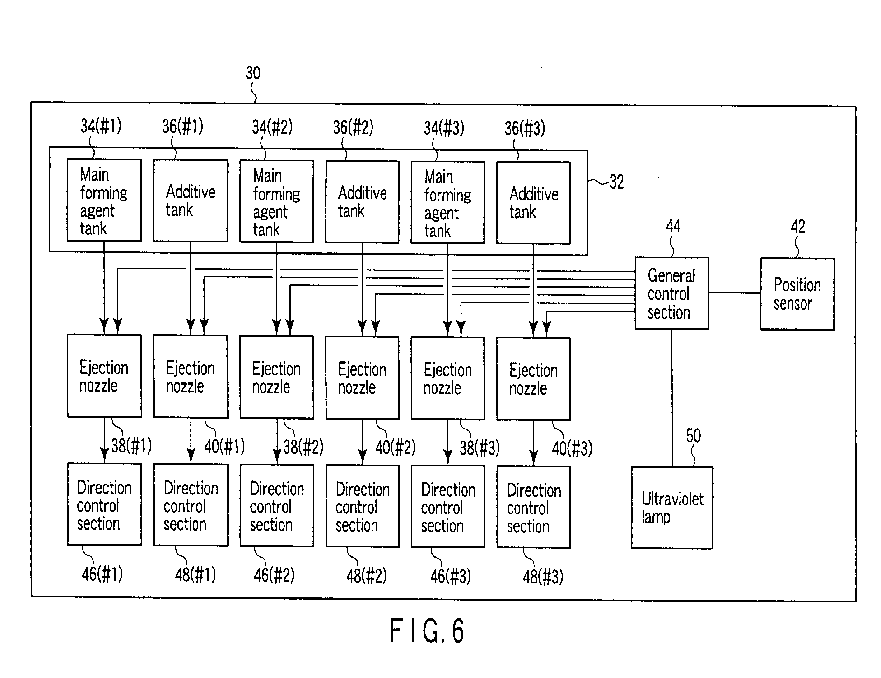

FIG. 6 is a functional block diagram showing an example of the configuration of an apparatus 30 for forming a solder wicking prevention zone to which apparatus the method for forming a solder wicking prevention zone according to the present embodiment is applied, the apparatus being developed in order to form the solder wicking prevention zone 16. The apparatus 30 for forming a solder wicking prevention zone according to the present embodiment comprises a tank unit 32, a plurality of ejection nozzles 38 and 40, a position sensor 42, a general control section 44, direction control sections 46 and 48 provided for the ejection nozzles 38 and 40 so as to constitute the respective pairs, and an ultraviolet lamp 50.

The ultraviolet lamp 50 need not necessarily be integrated with the apparatus 30 for forming a solder wicking prevention zone but may be separately provided.

The interior of the tank unit 32 is divided into six partial tanks 34 and 36. Three of them are main forming agent tanks 34 (#1, #2, and #3), while the remaining three are additive tanks 36 (#1, #2, and #3). The number of main forming agent tanks 34 has only to be the same as that of additive tanks 36. In addition, the number is not limited to three. Furthermore, only the main forming agent tank 34 (#1) may be provided with the others comprising additive tanks. A plurality of additive components suitable for main forming agent components may be stored in these tanks.

The main forming agent is a material used to form the solder wicking prevention zone 16. The main forming agent may be any material that may be organic or inorganic provided that it resists plating and is not wet with the molten solder 14. However, it should be able to at least avoid clogging the ejection nozzles 38 and 40. Materials are not preferable which deteriorate over time when exposed to the air, thus causing the clogging. In this sense, it is very significant to separately store the main forming agent and the additive to suppress secular changes.

In view of these points, the main forming agent comprises a fluorine-based resin, an epoxy-based resin, a silicone-based resin, a ultraviolet cured resin, a polyimide resin, or the like. The additive may properly comprise a polymerization initiator for initiating the polymerization of the main forming agent, a viscosity regulator for adjusting the viscosity of the main forming agent, a coloring agent for coloring the main forming agent, or the like.

The main forming agent stored in the main forming agent tanks 34 (#1, #2, and #3) is ejected from respective ejection nozzles 38 (#1, #2, and #3) in accordance with control provided by the general control section 44. The additive stored in the additive tanks 36 (#1, #2, and #3) is also ejected from respective ejection nozzles 40 (#1, #2, and #3) in accordance with control provided by the general control section 44.

When the main forming agent is thus ejected from the ejection nozzles 38 (#1, #2, and #3), the corresponding direction control sections 46 (#1, #2, and #3) control the ejection direction of the main forming agent so that the ejected main forming agent is ejected in a predetermined direction. Similarly, when the additive is thus ejected from the ejection nozzles 40 (#1, #2, and #3), the corresponding direction control sections 48 (#1, #2, and #3) control the ejection direction of the additive so that the ejected additive is ejected in a predetermined direction. The method used by the direction control sections 46 and 48 to control the direction is based on a principle similar to the one applied to existing ink jet printers.

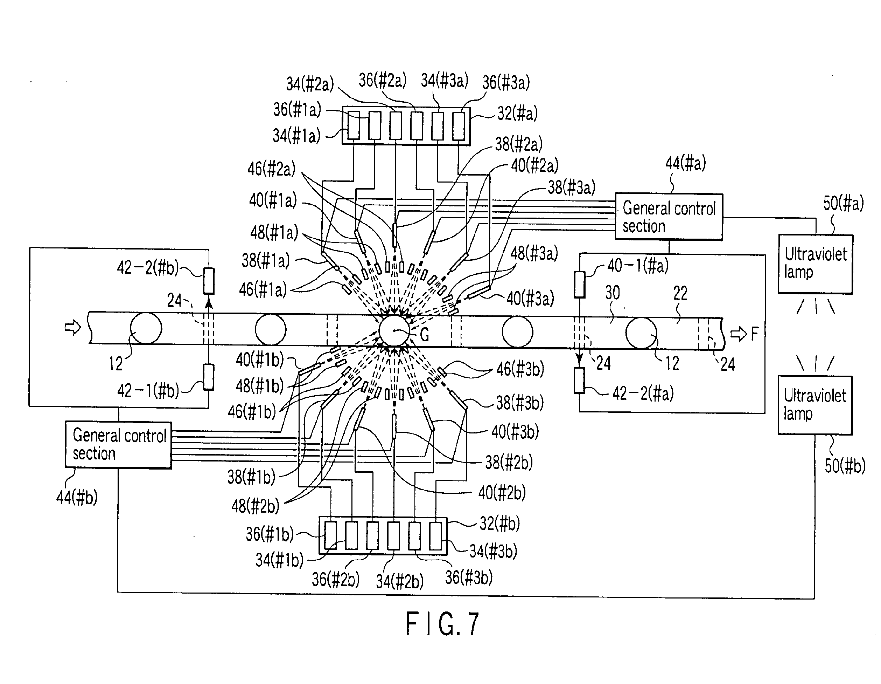

Upon sensing that the connector portion 12 is at a predetermined position, the position sensor 42 outputs a sense signal to the general control section 44. An example of this sensing method will be described later with reference to FIG. 7.

When the position sensor 42 outputs a sense signal, the general control section 44 allows the ejection nozzles 38 to eject the main forming agent, while allowing the ejection nozzles 40 to eject the additive. The general control section 44 further lights the ultraviolet lamp 50 to irradiate the corresponding connector portion 12 with ultraviolet rays.

FIG. 7 is a schematic view showing an example of arrangement of the components of the apparatus 30 for forming a solder wicking prevention zone. The illustrated connector portions 12 and planar portion 22 are viewed from the direction of the line A-A shown in FIG. 3. In FIG. 7, two apparatuses 30 (#a) and 30 (#b) for forming a solder wicking prevention zone according to the present embodiment are provided at the right and left, respectively, of the connector portions 12.

Reference character F, shown by an arrow in the figure, denotes a conveying direction F in which the connector portions 12 are conveyed together with the planar portion 22 using conveying means (not shown). The main forming agent and the additive are generally immiscible with water. Accordingly, the solder wicking prevention zone of the connector portion 12 is dried in advance.

Then, if a target center G coincides with the central axis of the connector portion 12, the position sensor 42 senses this and outputs a sense signal to the general control section 44. The position sensor 42 is composed of, for example, a photodiode 42-1, and a light receiving sensor 42-2. Pre-adjustment is made so that if the target center G coincides with the central axis of the connector portion 12, light emitted by the photodiode 42-1 passes through a positioning hole 24 and is received by the light receiving sensor 42-2. Upon receiving the light emitted by the photodiode 42-1, the light receiving sensor 42-2 outputs a sense signal.

When the position sensor 42 (#a) outputs the sense signal, the general control section 44 (#a) allows the ejection nozzles 38 (#1a, #2a, and #3a) to eject the main forming agent, while allowing the ejection nozzles 40 (#1a, #2a, and #3a) to eject the additive. The general control section 44 (#a) further lights the ultraviolet lamp 50 (#a) to irradiate the connector portion 12 with ultraviolet rays.

Likewise, when the position sensor 42 (#b) outputs the sense signal, the general control section 44 (#b) allows the ejection nozzles 38 (#1b, #2b, and #3b) to eject the main forming agent, while allowing the ejection nozzles 40 (#1b, #2b, and #3b) to eject the additive. The general control section 44 (#b) further lights the ultraviolet lamp 50 (#b) to irradiate the connector portion 12 with ultraviolet rays.

The ejection nozzles 38 (#1a, #2a, and #3a) and ejection nozzles 40 (#1a, #2a, and #3a) in the apparatus 30 (#a) for forming a solder wicking prevention zone as well as the ejection nozzles 38 (#1b, #2b, and #3b) and ejection nozzles 40 (#1b, #2b, and #3b) in the apparatus 30 (#b) for forming a solder wicking prevention zone are arranged at almost equal intervals so as to surround the target center G.

The direction control sections 46 (#1a, #2a, and #3a) corresponding to the ejection nozzles 38 (#1a, #2a, and #3a) and the direction control sections 46 (#1b, #2b, and #3b) corresponding to the ejection nozzles 38 (#1b, #2b, and #3b) make adjustment such that the ejected main forming agent is applied so as to cover the outer periphery of the connector portion 12 placed so that its central axis coincides with the target center G.

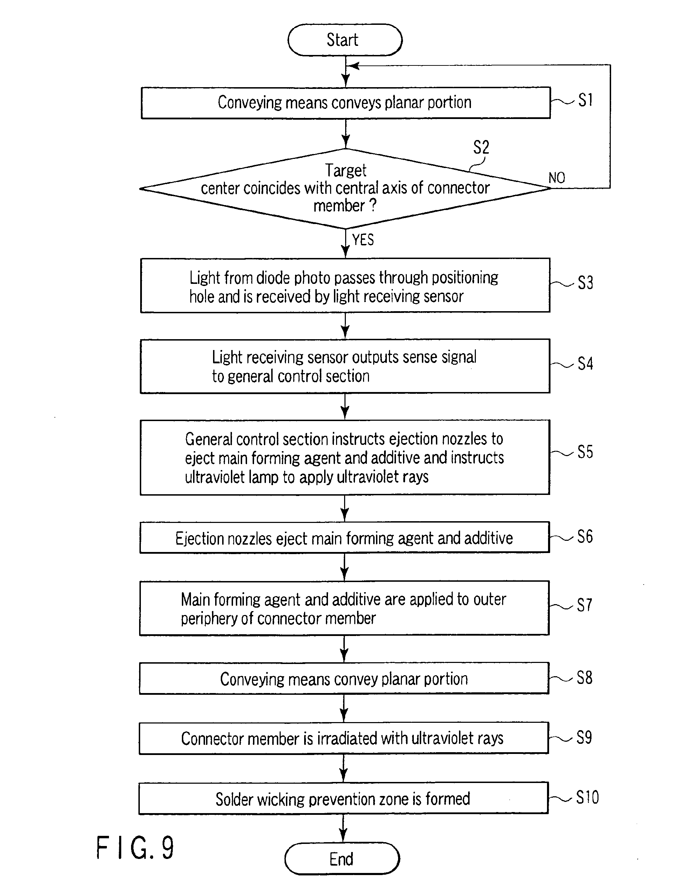

Specifically, if there are six ejection nozzles 38 from which the main forming agent is ejected (the ejection nozzles 38 (#1a, #2a, #3a, #1b, #2b, and #3b)), each direction control section 46 makes adjustment such that the main forming agent is applied in a sharable manner to a part R of the outer periphery of the connector portion which part has an angle θ of at least 60° around the target center G as shown in FIG. 8. Thus, the main forming agent ejected through the six ejection nozzles is applied so as to cover the entire outer periphery of the connector portion 12.

Likewise, the directional control sections 48 (#1a, #2a, and #3a) corresponding to the ejection nozzles 40 (#1a, #2a, and #3a) and the directional control sections 48 (#1b, #2b, and #3b) corresponding to the ejection nozzles 40 (#1b, #2b, and #3b) make adjustment such that the ejected main forming agent is applied so as to cover the outer periphery of the connector portion 12 placed so that its central axis coincides with the target center G.

Specifically, if there are six ejection nozzles 40 from which the additive is ejected (the ejection nozzles 40 (#1a, #2a, #3a, #1b, #2b, and #3b)), each direction control section 48 makes adjustment such that the main forming agent is applied in a sharable manner to the part R of the outer periphery of the connector portion which part has an angle θ of at least 60° around the target center G as shown in FIG. 8. Thus, the additive ejected through the six ejection nozzles is applied so as to cover the entire outer periphery of the connector portion 12.

In FIG. 7, each apparatus 30 for forming a solder wicking prevention zone comprises the three ejection nozzles 38 and the three ejection nozzles 40. However, the numbers of the ejection nozzles 38 and 40 are not limited to this aspect. However, if the numbers are smaller, the main forming agent or additive ejected from each ejection nozzle 38 or 40, respectively, must be ejected over a larger angle. This not only makes the ejection unstable but also increases the amount of main forming agent and additive which fail to be applied to the connector portion 12. Consequently, the main forming agent and the additive are inefficiently applied. Further, with an increase in the number of the ejection nozzles 38 and 40, it is more difficult to arrange these nozzles 38 and 40 owing to limited spaces. Furthermore, the whole configuration of the apparatus becomes complicated to make maintenance or the like more difficult. Therefore, the numbers of the ejection nozzles 38 and 40 are determined taking these points into account.

Now, description will be given of operations of the apparatus 30 for forming a solder wicking prevention zone, the components of which are arranged as shown in FIG. 7.

First, the planar portion 22 is conveyed along the conveying direction F, shown in FIG. 7, using conveying means (not shown) (S1). If the target center G coincides with the central axis of any connector portion 12 (S2: Yes), light emitted by the photodiodes 42-1 (#a and #b) passes through the positioning hole 24 and is the received by the light receiving sensors 42-2 (#a and #b) (S3). Then, the light receiving sensors 42-2 (#a and #b) output sense signals to the general control sections 44 (#a and #b), respectively (S4).

Upon acquiring the sense signal, the general control section 44 (#a) instructs the ejection nozzles 38 (#1a, #2a, and #3a) and the ejection nozzles 40 (#1a, #2a, and #3a) to eject the main forming agent and the additive. The general control section 44 (#a) also instructs the ultraviolet lamp 50 (#a) to irradiate the connector portion. Similarly, upon acquiring the sense signal, the general control section 44 (#b) instructs the ejection nozzles 38 (#1b, #2b, and #3b) and the ejection nozzles 40 (#1b, #2b, and #3b) to eject the main forming agent and the additive. The general control section 44 (#b) also instructs the ultraviolet lamp 50 (#b) to irradiate the connector portion (S5).

In response to this, the main forming agent is emitted from the six ejection nozzles 38 (#1a, #2a, #3a, #1b, #2b, and #3b), arranged to surround the connector portion 12 placed so that its central axis coincides with the target center G. The additive is emitted from the six ejection nozzles 40 (#1a, #2a, #3a, #1b, #2b, and #3b), arranged to surround the connector portion 12 placed so that its central axis coincides with the target center G (S6).

The main forming agent ejected from each of the ejection nozzles 38 (#1a, #2a, #3a, #1b, #2b, and #3b) has its ejection direction controlled by the corresponding control section 46 (#1a, #2a, #3a, #1b, #2b, and #3b). As a whole, the main forming agent is applied so as to cover the entire outer periphery of the connector portion 12. Likewise, the additive ejected from each of the ejection nozzles 40 (#1a, #2a, #3a, #1b, #2b, and #3b) has its ejection direction controlled by the corresponding control section 48 (#1a, #2a, #3a, #1b, #2b, and #3b). As a whole, the additive is applied so as to cover the entire outer periphery of the connector portion 12 (S7).

Thus, the main forming agent and the additive are applied so as to cover the entire outer periphery. Then, when the planar portion 22 is conveyed in the conveying direction F, shown in FIG. 7, using the conveying means (not shown) (S8), the next connector portion 12 is conveyed to the target center G. Then, the main forming agent and the additive are similarly applied to the connector portion 12.

The connector portion 12 coated with the main forming agent and additive is conveyed to the vicinity of the ultraviolet lamps 50 (#a and #b) as the planar portion 22 is conveyed. Then, the connector portion 12 is irradiated with ultraviolet rays emitted by the ultraviolet lams 50 (#a and #b) (S9). Thus, if the main forming agent is a ultraviolet cured resin, it is effectively hardened to stably form the solder wicking prevention zone 16 (S10). The solder wicking prevention zone 16 may be peeled off after the molten solder 14 has been applied or may constitute a final product without being further processed, as required.

The above solder wicking prevention zone 16 may be formed in any step provided that its purpose can be achieved. Accordingly, the solder wicking prevention zone 16 may be formed before nickel plating rather than after the connector portion 12 has been coated with the nickel plating 18.

In the description of the above example, the planar portion 22 is intermittently conveyed using the conveying means (not shown). However, the present invention is not limited to the intermittent conveyance of the planar portion 22 using the conveying means. The planar portion 22 may be continuously conveyed.

As is well known, ink jet printers represent an advanced technique to simultaneously use plural types of ink to accomplish high-quality color printing. It is adequately possible to carry out continuous printing while precisely controlling a line width to at most 0.5 mm. Additionally, the line width can be instantaneously set at an arbitrary value on the order of several dozen µm to several cm at an arbitrary time. This provides a high degree of freedom compared to the prior art.

Owing to the use of the principle of ink jet printers, the connector portions are not exposed to high temperature as in the case of a laser beam. The base material 20 and the plated surface are not impaired. Consequently, the present invention can be favorably used for the high quality connector potions 12, the pitch of which has been rapidly reduced.

Moreover, the time required to switch production can be sharply reduced. This makes it possible to improve productivity, while reducing costs.

Further, the ink jet printer can simultaneously inject plural types of ink and precisely control the injection. The ink jet printer can thus execute printing on a site such as a concave portion of a curved body or a press ruptured surface on which the prior art fails to achieve printing. Accordingly, for example, the solder wicking prevention zone 16 can be formed by individually storing the main forming agent and the additive (polymerization initiator, polymerization regulator, viscosity regulator, coloring agent, or the like) and simultaneously or individually ejecting them, as described above. The main forming agent and the additive need not be prepared before the formation of the solder wicking prevention zone 16. It is thus possible to suppress secular changes in main forming agent or additive. This means that the properties of the solder wicking prevention zone 16 can always be maintained and that the solder wicking prevention zone 16 is thus very reliable.

Thus, with the apparatus for forming a solder wicking prevention zone to which apparatus the method of forming a solder wicking prevention zone according to the present embodiment is applied, it is possible to stably form the solder wicking prevention zone 16 by using the principle of ink jet printers to apply the main forming agent and the additive as required.

### (Second Embodiment)

A second embodiment of the present invention will be described below with reference to the drawings.

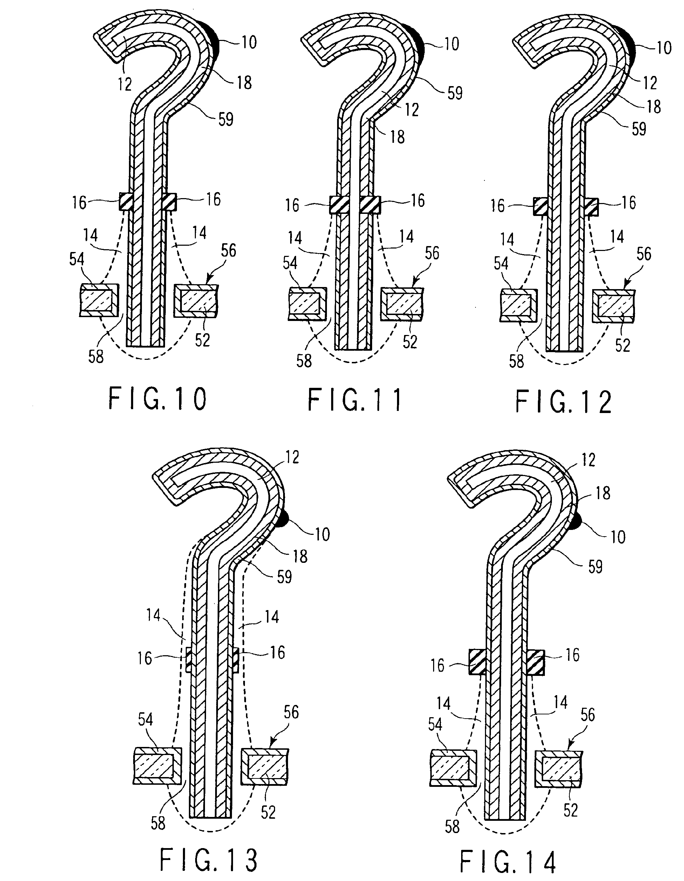

FIG. 10 is a sectional view showing another example of a connector member comprising a solder wicking prevention zone. Now, with reference to FIG. 10, description will be given of the case in which the connector portion 12 is inserted into a through-hole 58 in a printed circuit board 56 composed of a glass epoxy base material 52 the periphery of which is coated with copper plating 54, the connector portion 12 being then fixed using flow solder.

Specifically, an apparatus for forming a solder wicking prevention zone to which apparatus a method for forming a solder wicking prevention zone according to the present embodiment is applied forms the solder wicking prevention zone 16 that operates if the molten solder 14 is applied to an electronic part such as the connector portion 12 comprising the gold plated contact site 10 provided on, for example, gold flash plating 59, to hinder the molten solder 14 from rising and spreading to the gold plated contact site 10 in a wettable manner, as shown in FIG. 10. The connector portion 12 is composed of, for example, a copper alloy and is entirely coated with nickel plating 18.

The solder wicking prevention zone formed by the apparatus for forming a solder wicking prevention zone to which apparatus the method for forming a solder wicking prevention zone according to the present embodiment is applied is not limited to the one provided on the surface coated with the nickel plating 18 as shown in FIG. 10. The solder wicking prevention zone 16 may be provided directly on the material as shown in FIG. 11. Alternatively, the solder wicking prevention zone 16 may be provided on the gold flash plating 59. Alternatively, thick gold plating may be used in place of the gold flash plating 59.

If the provided solder wicking prevention zone 16 must have its width reduced to at most 0.5 mm, when the molten solder is applied, the solder may spread over the solder wicking prevention zone 16 toward the gold plated contact site 10 as shown in FIG. 13. To prevent this, it is effective to increase the height of the solder wicking prevention zone 16. In this case, it is advantageous to utilize a method of forming a solder wicking prevention zone using a dispenser as described later. This is because this method allows a forming agent that is more viscous (contains more effective components) than that used in the ink jet method described in the first embodiment.

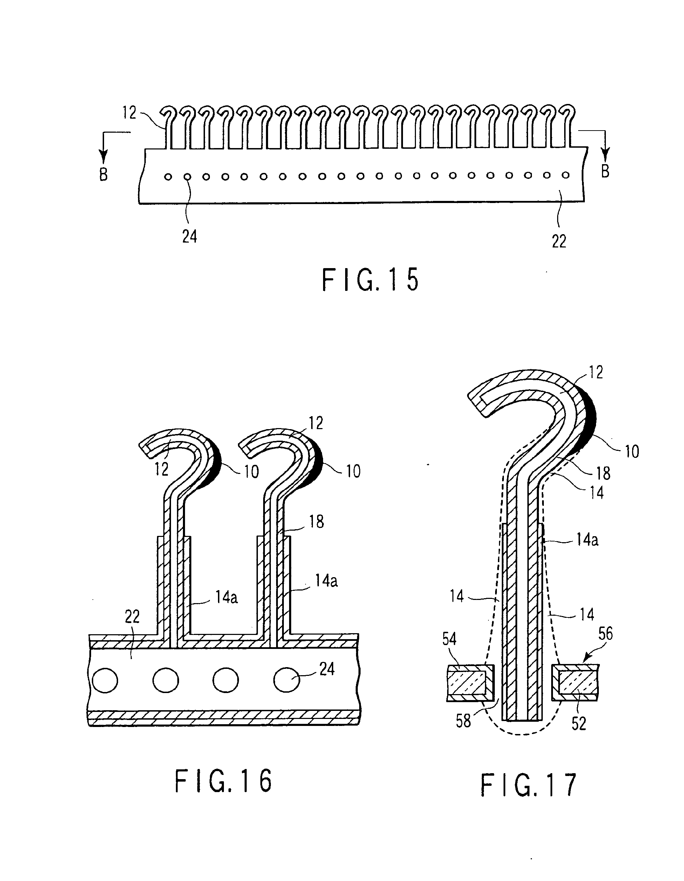

As an example of a method for forming an electronic member, with reference to FIGS. 2 and 15, description will be given of a method for forming the connector portion 12.

For each connector portion 12, the base material 20 composed of, for example, a copper alloy, such as the one shown in FIG. 2, is pressed into a shape such as the one shown in FIG. 15. The positioning holes 24 are drilled, at a fixed pitch, in the planar portion 22 on which the connector portions 12 have not been formed.

Then, as shown in FIG. 16, the nickel plating 18 is applied so as to cover all the surfaces of the connector portion 12 and planar portion 22. Moreover, the connector portion 12 is partly plated with gold to form the gold plated contact site 10. A leg portion of the connector portion 12 is plated with preliminary solder 14a. Subsequently, the connector portion 12 is cut off from the planar portion 22. The molten solder 14 is then used to integrate the preliminary solder 14a portion with another part to join the connector portion 12 to this part.

On this occasion, without the solder wicking prevention zone 16, the molten solder 14 may spread to the height of the gold plated contact site 10 in a wettable manner. Consequently, the connector portion may not function as a contact.

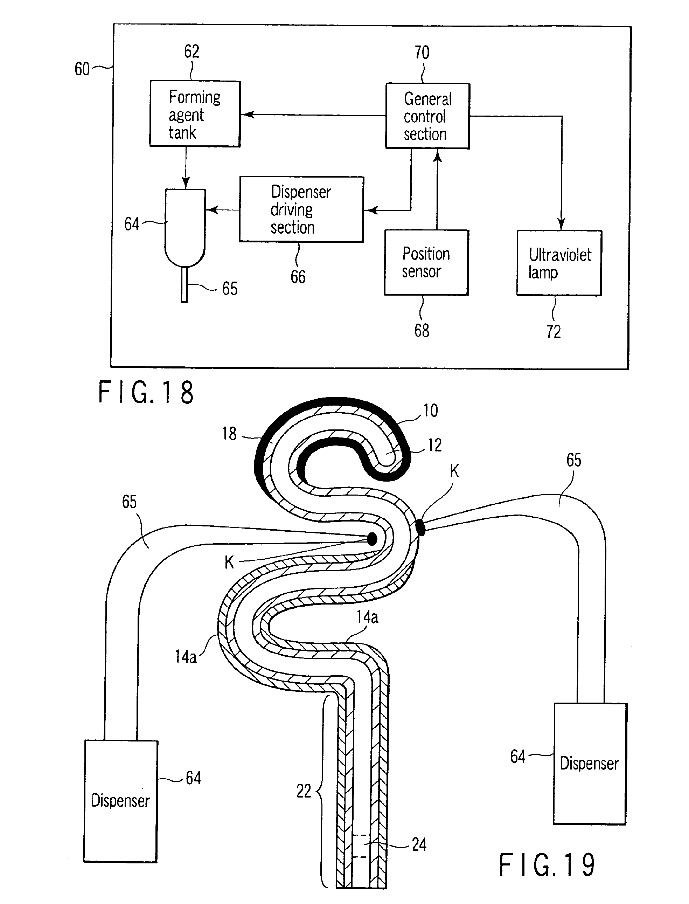

FIG. 18 is a functional block diagram showing an example of the configuration of an apparatus 60 for forming a solder wicking prevention zone to which apparatus the method for forming a solder wicking prevention zone according to the present embodiment is applied, the apparatus being developed in order to form the solder wicking prevention zone 16. The apparatus 60 for forming a solder wicking prevention zone according to the present embodiment comprises a forming agent tank 62, a dispenser 64, a dispenser driving section 66, a position sensor 68, a general control section 70, and an ultraviolet lamp 72.

The ultraviolet lamp 72 is used only if a ultraviolet cured resin is used as the forming agent. If the forming agent is different from the ultraviolet cured resin, the curing method is changed to one suitable for the curing of the forming agent used. Further, the ultraviolet lamp 72 need not necessarily be integrated with the apparatus 60 for forming a solder wicking prevention zone but may be separately provided. It is also unnecessary to integrate the ultraviolet lamp 72 with the apparatus 60 if a different curing method is used.

The forming agent tanks 62 internally store the forming agent, used to form the solder wicking prevention zone 16. The viscosity of the forming agent may be arbitrarily selected: the forming agent may have either a low viscosity of at most 1(Pa·s) or a high viscosity corresponding to a grease-like agent. It is possible to select a material that is suitable for the solder wicking prevention zone 16 and that has the optimum viscosity for the film thickness corresponding to the performance required for the solder wicking prevention zone 16. Such a material contains at least one or more of, for example, resin, preferably a ultraviolet cured resin, ceramic, carbon, and glass. It may also contain a water- or oil-repellent material such as a flux oozing preventing agent. A coloring agent for coloring the solder wicking prevention zone 16 may be properly mixed with the forming agent. It is then possible to easily recognize that the solder wicking prevention zone 16 is formed on the connector portion 12 and to easily distinguish the connector portion 12 from other parts.

In accordance with control provided by the general control section 70, a specified amount of forming agent stored in the forming agent tank 62, such as that described above, is ejected from the top of a nozzle 65 of the corresponding dispenser 64 at a specified ejection speed.

Upon sensing that a forming agent K is at a position where it can be applied, the position sensor 68 outputs a sense signal to the general control section 70. An example of this sensing method will be described later.

When the position sensor 68 outputs the sense signal, the general control section 70 transmits a signal to the dispenser driving section 66 to set the tip of the nozzle 65 at a predetermined position. The general control section 70 also transmits a signal to the forming agent tank 62 to subject the forming agent tank 62 to a pressure predetermined depending on the nature of the stored forming agent, the material of the connector portions 12, and the width and thickness of the solder wicking prevention zone 16 to be formed. This pressure causes the forming agent stored in the forming agent tank 62 to be pushed out into the dispenser 64. Then, a predetermined amount of forming agent is ejected from the tip of the nozzle 65 at a predetermined speed. The setting of the tip of the nozzle 65 at the predetermined position may be disconnected from and be independent of the general control section 70 or may be manually carried out.

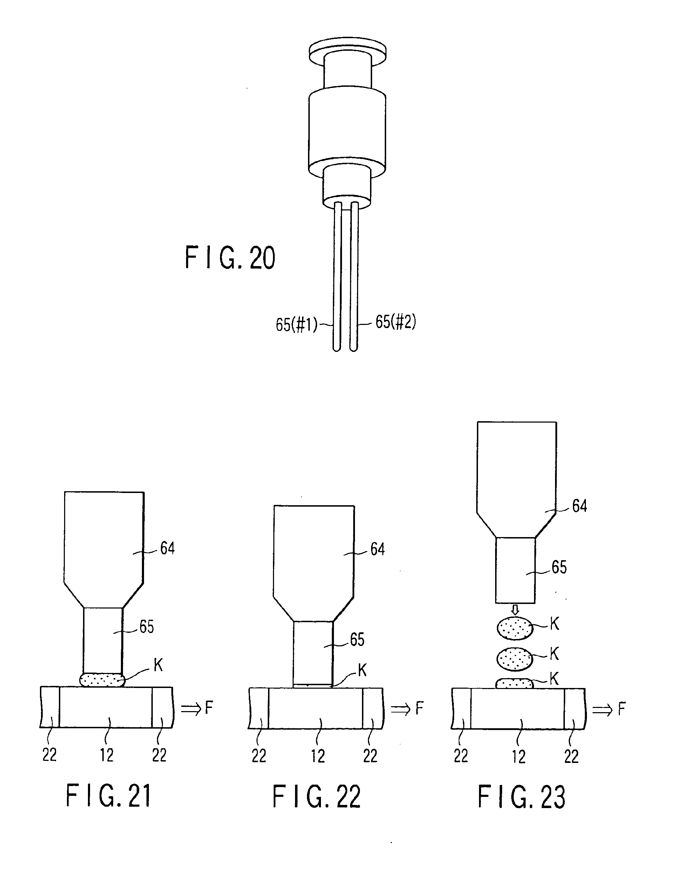

Many types of nozzles made of metal or resin are commercially available. Any of them may be selected as the nozzle 65 depending on the application. The direction of the tip of the nozzle 65 is pre-adjusted so that the ejected forming agent is applied to a predetermined position on the connector portion 12. The tip of the commercially available nozzle 65 can be thinned, elongated, or bent. Accordingly, as shown in FIG. 19, it is possible to alter the shape of the nozzle 65 in accordance with the configuration of the apparatus and to install the tip of the nozzle 65 in association with the predetermined position on the connector portion 12. Furthermore, as shown in FIG. 20, a multi-type nozzle 65 (#1 and #2) is commercially available which has a plurality of ejection openings through which the forming agent is ejected to the respective points.

The bore of the tip of the nozzle 65 determines the width of the solder wicking prevention zone 16 to be formed. Accordingly, the tip of the nozzle should have the appropriate size depending on the width of the solder wicking prevention zone 16 to be formed. The nozzle 65 is commercially available and can be selected to have an arbitrary size between 0.1 and several mm in diameter. That is, the nozzle 65 can be very freely selected depending on the application. In addition, the nozzle 65 is inexpensive.

The positional relationship between the nozzle 65 and the connector portion 12 may be such that the tip of the nozzle 65 is slightly separated from the surface of the connector portion 12 as shown in FIG. 21 or is pressed against the surface of the connector portion 12 to the degree that the connector portion 12 is not deformed as shown in FIG. 22. Alternatively, as shown in FIG. 23, the tip of the connector 65 may be completely separated from the surface of the connector portion 12. The connector portion 12 and planar portion 22 shown in FIGS. 21, 22, and 23 are viewed from the direction of the line B-B shown in FIG. 15.

In FIG. 21, the forming agent K ejected from the tip of the nozzle 65 grows into a droplet before being applied to the connector portion 12. Furthermore, the tip of the nozzle 65 does not come into contact with the connector portion 12. Consequently, the connector portion 12 is not damaged.

In FIG. 22, the forming agent K ejected from the tip of the nozzle 65 is applied directly to the connector portion 12 before growing into a droplet.

In FIG. 23, the forming agent K ejected from the tip of the nozzle 65 is shot so as to be applied to the connector portion 12. In this case, the application can be controlled in a short time on the order of milliseconds per shot. Furthermore, the forming agent can be precisely shot over an ejection distance of about 10 mm. Accordingly, this method is suitable for the connector portion 12 that is relatively easily deformed or that cannot be approached by the nozzle 65. To form the solder wicking prevention zone 16 on the connector portion 12, particularly on a press ruptured surface, it is necessary to incline the tip of the nozzle 65 from the target position.

The above appropriate placement of the nozzle 65 enables the forming agent K to be reliably applied to an arbitrary predetermined position on the connector portion 12, which tends to be further miniaturized and complicated. Moreover, a plurality of solder wicking prevention zones 16 can be easily provided on the connector portion 12 in order to enhance the prevention of solder wicking.

If a ultraviolet cured resin is used as the forming agent K, the ultraviolet lamp 50 irradiates the ultraviolet cured resin applied to the connector portion with ultraviolet rays to harden the resin.

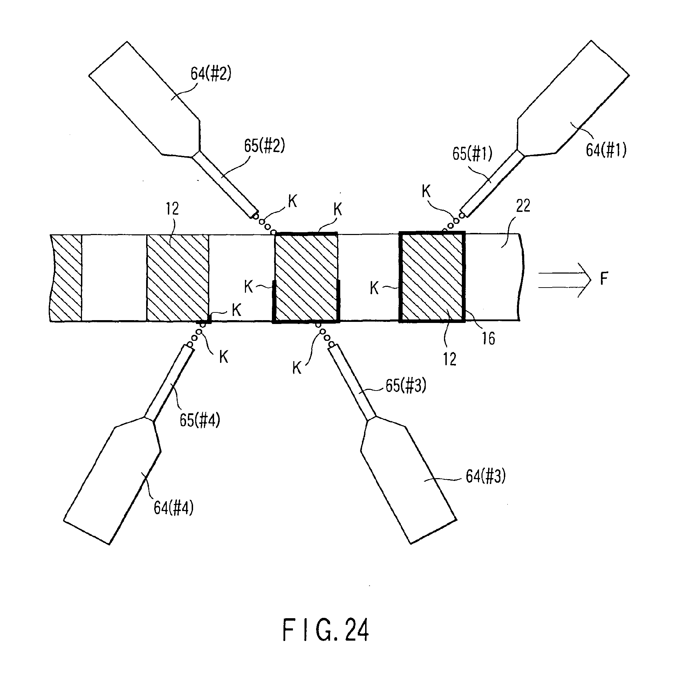

FIG. 24 is a schematic diagram showing an example of arrangement of the components of the above described apparatus 30 for forming a solder wicking prevention zone. The illustrated connector portions 12 and planar portion 22 are viewed from the line B-B shown in FIG. 15. FIG. 24 shows the example in which the apparatus comprises a pair of dispensers 64 (#1 and #2) arranged at one side of the planar portion and a pair of dispensers 64 (#3 and #4) arranged at the other side. A nozzle 65 (#1) of the dispenser 64 (#1) and a nozzle 65 (#4) of the dispenser 64 (#4) are arranged in opposite directions. A nozzle 65 (#2) of the dispenser 64 (#2) and a nozzle 65 (#3) of the dispenser 64 (#3) are arranged in opposite directions. In this manner, a plurality of dispensers 64 and a plurality of nozzles 65 may be provided. Then, the forming agent K ejected from the nozzles 65 (#1 and #2) to one half of the outer periphery of each connector portion 12 as shown in FIG. 24. The forming agent K ejected from the nozzles 65 (#3 and #4) is applied to the remaining half of the outer periphery of each connector portion 12. The solder wicking prevention zone 16 is thus formed. Of course, if the base material 20 is thin, only one dispenser 64 and only one nozzle 65 may be provided at each side.

If the forming agent K is shot so as to be applied to the connecting portion 12, the pair of nozzles 65 (#1 and #2) and the pair of nozzles 65 (#3 and #4) are arranged obliquely to the conveying direction and opposite each other.

The apparatus 60 for forming a solder wicking prevention zone comprises conveying means (not shown). The conveying means continuously or intermittently conveys the connector portions 12 along the conveying direction F together with the planar portion 22.

As shown in FIG. 24, if the conveying means (not shown) conveys the connector portions 12 along the conveying direction F and one of the connector portion 12 reaches a point closest to each of the nozzles 65 (#1, #2, #3, and #4), then the position sensor 68 senses this and outputs a sense signal to the general control section 70. The position sensor 68 may be a combination of a photodiode and a light receiving sensor or may use a method such as image processing.

When the position sensor 68 thus outputs the sense signal, the general control section 70 transmits a signal to the dispenser driving section 66 to set the tip of each nozzle 65 at a predetermined position. The general control section 70 also transmits a signal to each of the forming agent tanks 62 to subject the forming agent tank 62 to a pressure predetermined depending on the nature of the stored forming agent, the material of the connector portions 12, and the width and thickness of the solder wicking prevention zone 16 to be formed. This pressure causes the forming agent K stored in each forming agent tank 62 to be pushed out into the corresponding dispenser 64. Then, a predetermined amount of forming agent K is ejected from the tip of each of the nozzles 65 at a predetermined speed.

The conveying means conveys, along the conveying direction F, the connector portions 12 with the forming agent K applied to the predetermined position. The ultraviolet lamp 72 is provided at the leading end in the conveying direction. The forming agent K applied to the connector portion 12 is irradiated with ultraviolet rays emitted by the ultraviolet lamp 72. Thus, if the forming agent is a ultraviolet cured resin, it is efficiently hardened. If the forming agent is a material different from the ultraviolet cured resin, the ultraviolet lamp 72 is unnecessary. However, in this case, the curing method is changed to one suitable for the curing of the forming agent used.

Now, description will be given of operations of the apparatus 60 for forming a solder wicking prevention zone, such as the one shown in FIG. 18.

First, if the conveying means (not shown) conveys the connector portions 12 along the conveying direction F and one of the connector portion 12 reaches a point closest to each nozzle 65, then the position sensor 68 senses this. The position sensor 68 then outputs a sense signal to the general control section 70.

Upon receiving this sense signal, the general control section 70 transmits a signal to the dispenser driving section 66 to set the tip of each nozzle 65 at a predetermined position. Simultaneously, each of the forming agent tanks 62 is pressurized. The value of this pressure is predetermined depending on the nature of the forming agent K stored in each forming agent tank 62, the material of the connector portions 12, and the width and thickness of the solder wicking prevention zone 16 to be formed.

This pressure causes the forming agent K stored in each forming agent tank 62 to be pushed out into the corresponding dispenser 64. Then, a predetermined amount of forming agent K is ejected from the tip of each of the nozzles 65 at a predetermined speed.

Thus, the forming agent K is applied to the entire periphery of the connector portion 12 as shown in FIG. 24.

In this manner, the solder wicking prevention zone 16 is reliably formed along the entire periphery of a predetermined position of the connector portion 12. Further, the dispensers 64 and the nozzles 65 enable the use of a forming agent that is more viscous than that used in the prior art. It is thus also possible to provide a more stable solder wicking prevention zone 16 using such a more viscous forming agent. Moreover, by pre-mixing the forming agent K with a coloring agent, it can be easily recognized that the connector portion 12 is formed with the solder wicking prevention zone 16. Furthermore, coloring the solder wicking prevention zone allows electronic parts to be easily discriminated from each other.

The solder wicking prevention zone 16 may be peeled off after the preliminary solder 14a has been provided or may constitute a final product without being further processed, as required. The above solder wicking prevention zone 16 may be formed in any step provided that its purpose can be achieved. Accordingly, the solder wicking prevention zone 16 may be formed before nickel plating rather than after the connector portion 12 has been coated with the nickel plating 18. Alternatively, the solder wicking prevention zone 16 may be formed after gold plating following the nickel plating.

As described above, with the apparatus for forming a solder wicking prevention zone to which apparatus the method for forming a solder wicking prevention zone according to the present embodiment is applied, it is ensured that the solder wicking prevention zone 16 can be efficiently and stably formed precisely along the outer periphery of the predetermined position of the connector portion. Moreover, by pre-mixing the forming agent K with a coloring agent, it can be easily recognized that the connector portion 12 is formed with the solder wicking prevention zone 16.

Additionally, many types of nozzles made of metal or resin are commercially available. Any of them may be selected as the nozzle 65 depending on the application. Additionally, the shape of the nozzle 65 may be thinned, elongated, or bent in accordance with the configuration of the apparatus. Accordingly, the tip of the nozzle 65 can be easily fixed at the predetermined position relative to the connector portion. This serves to avoid extra costs to enable the apparatus to be inexpensively constructed.

With reference to the accompanying drawings, the preferred embodiments of the present invention have been described in conjunction with the connector portion 12. However, the present invention is not limited to such configurations.

## Claims

1. An apparatus (30) for forming a solder wicking prevention zone (16) operating when a connector member with a connector is processed using molten solder (14), to hinder the solder from spreading to a contact site (10) of the connector (12) in a wettable manner, the apparatus **characterized by** comprising:
storage means (34) for storing a forming agent used to form the solder wicking prevention zone;
ejecting means (38) for ejecting the forming agent stored in the storage means; and
control means (46) for controlling an ejection direction of the forming agent ejected by the ejecting means,
wherein the control means controls the ejection direction of the forming agent ejected by the ejecting means, so as to apply the forming agent to a predetermined position on the connector member to form the solder wicking prevention zone.

2. The apparatus (30) according to claim 1, **characterized in that** a plurality of the ejecting means (38) and a plurality of the corresponding control means (46) are arranged to surround the predetermined position on the connector member so that the control means (46) corresponding to each ejecting means can control the ejection direction of the forming agent ejected by the ejecting means, thus allowing the forming agent to be applied to the predetermined position on the connector member.

3. The apparatus according to claim 1 or 2, **characterized in that** an ultraviolet cured resin is used as the forming agent, and
the apparatus further comprises ultraviolet irradiation means (50) for irradiating the predetermined position coated with the forming agent with ultraviolet rays.

4. An apparatus for forming a solder wicking prevention zone operating when a connector member with a connector is processed using molten solder, to hinder the solder from spreading to a contact site of the connector in a wettable manner, the apparatus **characterized by** comprising:
first storage means (34) for storing a main forming agent used to form the solder wicking prevention zone;
second storage means (36) for storing an additive added to the main forming agent;
first ejecting means (38) for ejecting the main forming agent stored in the first storage means;
second ejecting means (40) for ejecting the additive stored in the second storage means; and
control means (46, 48) for controlling ejection directions of the forming agent and additive ejected by the first and second ejecting means, respectively,
wherein the control means control the ejection directions of the forming agent and additive ejected by the first and second ejecting means, respectively, so as to apply the forming agent and the additive to a predetermined position on the connector member to form the solder wicking prevention zone.

5. The apparatus according to claim 4, **characterized in that** a plurality of the ejecting means and a plurality of the corresponding control means are arranged to surround the predetermined position on the connector member so that the control means corresponding to the respective ejecting means can control the ejection directions of the forming agent and additive ejected by the ejecting means, thus allowing the forming agent and the additive to be applied to the predetermined position on the connector member.

6. A method for forming a solder wicking prevention zone using an ink jet printer comprising storage means for storing ink, ejecting means for ejecting the ink stored in the storage means, and control means for controlling an ejection direction of ink ejected by the ejecting means, the solder wicking prevention zone operating when a connector member with a connector is processed using molten solder, to hinder the solder from spreading to a contact site of the connector in a wettable manner, the method **characterized by** comprising:
storing a forming agent used to form the solder wicking prevention zone, in the storage means, instead of the ink;
allowing the ejecting means to eject the forming agent stored in the storage means; and
allowing the control means to control an ejection direction of the forming agent ejected by the ejecting means, so as to apply the forming agent to a predetermined position on the connector member to form the solder wicking prevention zone.

7. The method according to claim 6, **characterized in that** a plurality of the ejecting means and a plurality of the corresponding control means are arranged to surround the predetermined position on the connector member so that the control means corresponding to each ejecting means can control the ejection direction of the forming agent ejected by the ejecting means, thus allowing the forming agent to be applied to the predetermined position on the connector member.

8. The method according to claim 6 or 7, **characterized in that** a ultraviolet cured resin is used as the forming agent, and the ultraviolet cured resin is solidified by irradiating the predetermined position coated with the forming agent with ultraviolet rays.

9. A method for forming a solder wicking prevention zone using an ink jet printer comprising first storage means for storing ink, second storage means for storing an additive added to the ink, first ejecting means for ejecting the ink stored in the first storage means, second ejecting means for ejecting the additive stored in the second storage means, and control means for controlling ejection directions of the ink and additive ejected by the first and second ejecting means, respectively, the solder wicking prevention zone operating when a connector member with a connector is processed using molten solder, to hinder the solder from spreading to a contact site of the connector in a wettable manner, the method **characterized by** comprising:
storing a main forming agent used to form the solder wicking prevention zone, in the first storage means, instead of the ink, and storing an additive added to the main forming agent, in the second storage means;
allowing the first ejecting means to eject the main forming agent stored in the first storage means;
allowing the second ejecting means to eject the additive stored in the second storage means; and
allowing the control means to control ejection directions of the forming agent and additive ejected by the first and second ejecting means, respectively, so as to apply the forming agent and the additive to a predetermined position on the connector member to form the solder wicking prevention zone.

10. The method according to claim 9, **characterized in that** a plurality of the ejecting means and a plurality of the corresponding control means are arranged to surround the predetermined position on the connector member so that the control means corresponding to the respective ejecting means can control the ejection directions of the forming agent and additive ejected by the ejecting means, thus allowing the forming agent and the additive to be applied to the predetermined position on the connector member.

11. An electronic part (12) comprising a solder wicking prevention zone (16) that hinders solder (14) from spreading in a wettable manner during a molten solder process, the solder wicking prevention zone being provided by applying a material that is not wet with the solder, to the electronic part.

12. The electronic part according to claim 11, **characterized in that** the material that is not wet with the solder contains at least one or more of resin, ceramic, carbon, and glass.

13. The electronic part according to claim 11 or 12, **characterized in that** the solder wicking prevention zone is colored differently from a coated surface so as to be discriminated from the coated surface.

14. An apparatus (60) for forming a solder wicking prevention zone operating when an electronic part is processed using molten solder, to hinder the solder from spreading out from a joined site of the electronic part in a wettable manner, the apparatus **characterized by** comprising:
storage means (62) for storing a forming agent used to form the solder wicking prevention zone;
ejecting means (64) for ejecting the forming agent stored in the storage means;
control means (70) for controlling an ejection amount and an ejection speed of the forming agent ejected by the ejecting means; and
curing means (72) for curing the forming agent ejected by the ejecting means and applied to a predetermined position on the electronic part.

15. The apparatus according to claim 14, **characterized in that** a plurality of the ejecting means are arranged to surround the predetermined position on the electronic part so that the control means can control the ejection amount and ejection speed of the forming agent ejected by each ejecting means, thus allowing the forming agent to be applied to the predetermined position.

16. The apparatus according to claim 14 or 15, **characterized in that** a ultraviolet cured resin is used as the forming agent, and the apparatus further comprises ultraviolet irradiation means for irradiating the forming agent applied to the predetermined position with ultraviolet rays.

17. The apparatus according to claim 14 or 15, **characterized in that** a dispenser (64) is used as the ejecting means.

18. The apparatus according to claim 16, **characterized in that** a dispenser (64) is used as the ejecting means.

19. A method for forming a solder wicking prevention zone operating when an electronic part is processed using molten solder, to hinder the solder from spreading out from a joined site of the electronic part in a wettable manner, the method **characterized by** comprising:
ejecting a predetermined amount of forming agent used to form the solder wicking prevention zone, from a nozzle of a dispenser at a predetermined ejection speed, to apply the forming agent to a predetermined position on the electronic part; and
curing the applied forming agent to form the solder wicking prevention zone.

20. The method according to claim 19, **characterized in that** a ultraviolet cured resin is used as the forming agent, and the forming agent applied to the predetermined position is irradiated with ultraviolet rays and thus hardened.
